# EUROPEAN PATENT APPLICATION

(11) **EP 2 085 465 A1**
(43) Date of publication of application: **05.08.2009**
(21) Application number: 07831982.9
(22) Date of filing: 16.11.2007
(51) Int. Cl.: C12M 3/00

(54) **TISSUE PIECE PINCHING DEVICE AND CULTURE KIT**

(30) Priority: 17.11.2006 JP 2006312136; 17.11.2006 JP 2006312137
(71) Applicant: Japan Tissue Engineering Co., Ltd., Gamagori-shi, Aichi 443-0022 (JP); Nipro Corporation, Kita-ku Osaka-shi, Osaka 531-8510 (JP)
(72) Inventor: YAMADA, Kazuto, Gamagori-shi Aichi 443-0022 (JP); IWATA, Naohito, Gamagori-shi Aichi 443-0022 (JP); KATO, Masakazu, Gamagori-shi Aichi 443-0022 (JP); HATA, Ken-Ichiro, Gamagori-shi Aichi 443-0022 (JP); SHIRASU, Akio, Osaka-shi Osaka 531-8510 (JP); YOSHIKAWA, Yoshihiro, Osaka-shi Osaka 531-8510 (JP); TAGUCHI, Atsushi, Osaka-shi Osaka 531-8510 (JP)
(74) Representative: Kuhnen & Wacker
(86) International application number: PCT/JP2007/072252
(87) International publication number: WO 2008/059945

(57) **Abstract**

Under a state where the zipper 22 of a culture bag is opened, an operator depresses the opening button 43 of an arm 40 with one finger. Consequently, a force for oscillating the arm 40 to separate a retaining portion 49 upward from the culture surface 32 around a pin 42 acts against the urging force of a leaf spring 50. Consequently, the tip 52a of an extended portion 52 ascends to a predetermined position while resisting against the urging force of the leaf spring 50. Since the lower surface 20a of the culture bag 20 is retained by a base 31 and the upper surface 20b is raised upward by the tip 52a of an extended portion 52, opening 24 of the culture bag 20 is widened. Since the width of the base 31 is larger than that of the arm 40, upper space of the culture surface 32 is not occupied by the arm 40 and a sufficiently wide work space is provided in the culture bag 20.

## Description

### TECHNICAL FIELD

The present invention relates to a tissue piece pinching device and a culture kit installed with the tissue piece pinching device.

### BACKGROUND ART

Recently, in accordance with an increase in usage of cultured tissues and cultured cell suspensions in regeneration medicine, culture kits suitable for obtaining culture tissues and cultured cell suspensions have been developed. For example, Patent Document 1 discloses a culture kit capable of quickly and easily forming a culture state suitable for culturing a tissue piece. Such culturing kit has a tissue piece pinching device arranged in a culture bag. The tissue piece pinching device pinches a tissue piece between a culture surface and a mesh sheet adherable to the culture surface. The culture bag has gas permeability and liquid impermeability, and includes an opening that is capable of being closed liquid-tight. By applying an external force from both ends of the opening toward the center, the opening opens and a space is formed between the culture surface and the mesh sheet. By releasing the external force, the opening is closed by its own restoring force, and the mesh sheet is adhered to the culture surface. Thus, when culturing a tissue piece, first, the opening is opened by applying an external force from both ends of the opening toward the center, and a space is formed between the culture surface and the mesh sheet. Then, the tissue piece is inserted through the opening and mounted on the culture surface. Thereafter, the applied external force is released so as to close the opening and the mesh sheet is adhered to the culture surface. Consequently, the tissue piece is pinched between the culture surface and the mesh sheet. Next, a liquid culture medium is injected into the culture bag and the gas inside the culture bag is discharged. Thereafter, the culture bag is placed in an incubator to culture the tissue piece. At this time, since the culture bag has gas permeability, the gas component in the incubator is dissolved in the liquid culture medium in the culture bag, and is supplied to the tissue piece. Also, Patent Document 2 discloses a fixture that is used in a rotary culture method. The fixture is structured so as to fix a tissue piece mounted on a glass plate by a fork-like band plate engaged by the glass plate, and is used for a rotary culture apparatus.
Patent Document 1: Japanese Patent Application Publication No. JP-A-2005-87029
Patent Document 2: Japanese Patent Application Publication No. JP-A-2003-61640

### DISCLOSURE OF THE INVENTION

However, the culture kit of Patent Document 1 has difficulty in its operation since an external force needs to be applied from both ends of the opening of the culture bag towards the center when opening the space between the culture surface and the mesh sheet. Also, the fixture of Patent Document 2 has difficulty in its operation, since, even though the fork-like band plate is slidably engaged so as to embrace the glass plate, the band plate cannot oscillate, and it is difficult to depress the tissue piece below a tip portion of the fork-like band plate.

In order to solve the problems described above, it is an object of the present invention to provide a tissue piece pinching device having favorable operability when handing a tissue piece and a cultured tissue. Another object is to provide a culture kit installed with such a tissue piece pinching device.

The present invention adopts the following means to achieve at least one of the above-mentioned objects.

A tissue piece pinching device capable of being arranged in a culture chamber according to the present invention includes: a base having a culture surface for mounting a tissue piece; an arm oscillatably attached to the base; and a retaining member, as a part of the arm, provided in a position capable of retaining the tissue piece mounted on the culture surface.

In the tissue piece pinching device, when mounting the tissue piece on the culture surface, the arm is oscillated so that the retaining member is separated from the culture surface. While keeping such a state, the tissue piece is mounted on the culture surface at a position facing the retaining member. To take out the cultured tissue, which is the cultured tissue piece, the arm is oscillated so that the retaining member is separated from the culture surface, and while keeping such a state, the cultured tissue is taken out from the culture surface. In this manner, when opening the space between the culture surface and the retaining member, the arm is oscillated. Consequently, the operation is preferable compared with applying an external force from both ends of the opening of the culture bag towards the center as in the related art.

Here, the culture chamber includes a culture cavity, and any structure may be adopted as long as the structure is capable of containing the tissue piece pinching device in the culture cavity, and for example, a petri dish, a flask, and a bag may be cited. Preferably, a flexible culture bag is used. The culture surface is not particularly limited as long as it is capable of culturing a cell composing the tissue piece, and for example, a culture surface formed by synthetic resin such as polyethylene may be cited. Also, the culture surface may be treated to add a hydrophilic property, or may be covered with a cell-adhesive material such as collagen and fibrin. As for the retaining portion, although any structure or material may be adopted as long as the tissue piece can be retained, the retaining portion preferably has a surface to which the cell is unlikely to adhere. As such a surface, for example, polyethylene, polypropylene, fluorine resin, polytetrafluoroethylene, polycarbonate, and polyester may be cited. Further, as for the tissue piece, epithelial tissue, connective tissue, muscular tissue, nerve tissue, or the like that can be taken from living tissue are preferable. Specifically, the skin (epidermis, dermis), cartilage, corneas, retinas, periostea, bones, nerves, muscles, mucosa, paradentium, blood vessels, adipo, or a part of an organ such as the heart, liver, pancreas, kidney, or bladder may be cited. Also, cultured tissue whose cell is seeded in a cell sheet obtained by culturing a cell or a scaffold may also be used as a tissue piece for the present invention. Such a cell can be taken from warm-blooded animals such as humans, mice, rats, guinea pigs, hamsters, chickens, rabbits, pigs, sheep, cows, horses, dogs, cats, and monkeys. As the cells of such warm-blooded animals, keratinized cells, splenic cells, nerve cells, glial cells, pancreatic β cells, mesangial cells, Langerhans cells, epidermal cells, epithelial cells, endothelial cells, fibroblast cells, fiber cells, muscle cells, adipose cells, synovial cells, cartilage cells, bone cells, periosteal cells, osteoblast cells, osteoclastic cells, breast cells, hepatic cells, stroma cells, or precursor cells, stem cells, or adhesion-dependent cancer cells of the above mentioned cells may be cited. Also, embryonic stem cells can be used. Also, transformed cells configured by introducing to the above-mentioned cells an exogenous gene that encodes erythropoietin, growth hormone, a granulocyte colony-stimulating factor, insulin, interferon, a blood coagulation factor such as a blood coagulation factor VIII, glucagons, tissue plasminogen activator, dopamine, cancer genes, cancer suppressor genes, or the like, and by expressing those genes forcibly by using a variety of promoters or under a certain condition. As for an extracellular matrix, integrin, collagen, elastin, proteoglycan, glycosaminoglycan, glycoprotein, or the like may be cited.

In the tissue piece pinching device according to the present invention, the width of the arm may be smaller than that of the base. Thus, the space over the culture surface is not occupied by the arm, and a sufficiently wide work space is provided in the culture chamber. Therefore, workability when handing the tissue piece and the cultured tissue becomes favorable.

The tissue piece pinching device according to the present invention may also include: an urging member that urges the retaining member towards the culture surface; and an arm operating member that is provided on the opposite side to the retaining member, with an oscillating shaft that is used when the arm oscillates interposed therebetween, and that separates, when depressed by an external force, the retaining member from the culture surface while resisting the urging member. In this case, in order to pinch the tissue piece, the arm operating member is depressed by applying an external force so as to separate the retaining member upward from the culture surface. Then, in such a state, the tissue piece is mounted on the culture surface. Thereafter, by releasing the external force applied to the arm operating member, the tissue piece mounted on the culture surface is depressed by the retaining member urged by the urging force. To take out the cultured tissue, which is the cultured tissue piece, the arm operating member is depressed by applying an external force so as to separate the retaining member upward from the culture surface. In such a state, the cultured tissue is taken out. In this manner, during culturing of the tissue piece, since the tissue piece is depressed against the culture surface by the retaining member urged by the urging member, the tissue piece adheres to the culture surface and becomes easy to grow. Also, the operation of the arm becomes easy. Particularly, when using a flexible culture bag as the culture chamber, since the operation can be made from the outside of the culture bag, contamination can be prevented. In the tissue piece pinching device according to this aspect of the present invention, the urging member may be formed integrally with the arm. In this manner, the number of components can be reduced compared with the case of forming the urging member and the arm separately, and the assembling process can be simplified.

The tissue piece pinching device according to the present invention may also include an extended member that extends from a position of the retaining member, as a part of the arm, in a direction opposite to the oscillating shaft used when the arm oscillates. Thus, workability becomes more favorable. For example, when using the tissue piece pinching device arranged in the flexible culture bag, the working space in the culture bag is secured by widening the inside of the culture bag by the arm oscillating so as to separate the retaining member from the culture surface. Here, when the arm oscillates so as to separate the retaining member from the culture surface, since the distance from the oscillating shaft to a tip end of the extended member is longer than the distance from the oscillating shaft to the retaining member, even when the depressing amount of the arm operating member is the same, thanks to the extended member, the working space becomes further larger and the operability becomes favorable compared to the case where there is no extended member. In the tissue piece pinching device according to this aspect of the present invention, the base may include a supporting member at a position capable of contacting a lower surface of a tip end side of the extended member. The supporting member prevents the extended member from rotating downward to exceed a predetermine range around the oscillating shaft of the arm. Even if a force that depresses the extended member is unexpectedly applied, the retaining member of the arm can be prevented from strongly depressing the culture surface. To put it another way, in a case where the base includes no supporting member, when a force that depresses the extended member from above is unexpectedly applied, the retaining member strongly depresses the culture surface, and there is a risk of damaging the tissue piece. Meanwhile, when the base includes the supporting member, since the supporting member supports the extended member from below, the retaining member does not strongly depress the culture surface. Also, the supporting member is preferably provided in a position deviated from the direct-front surface of the retaining member. With this arrangement, the supporting member will never be disturbing, for example, when mounting the tissue piece directly below the retaining member. Further, a supporting member that downwardly extends from the extended member or the arm may be provided, and by the supporting member contacting the culture surface of the base, rotation towards further below can be prevented.

In the tissue piece pinching device according to the present invention, the retaining member may be formed in such a shape that point-contacts or line-contacts the tissue piece mounted on the culture surface. Thus, compared with the retaining member formed in such a shape that plane-contacts the tissue piece, the tissue piece can be securely fixed to the culture surface, and the penetration (penetrativity) of the culture medium to the tissue piece becomes favorable.

In the tissue piece pinching device according to the present invention, the base may include a projection at least at a part of the peripheral of the culture surface. With this structure, when using the tissue piece pinching device arranged in the flexible culture bag, even in a case where the culture bag deflates by the culture medium being taken out from the culture bag for replacing the culture medium, the projection prevents the culture bag and the tissue piece from contacting with each other. Note that the projection may be a wall or a barrier that surrounds the entire periphery of the culture surface, or may be a wall or a barrier that partially surrounds the periphery of the culture surface.

In the tissue piece pinching device according to the present invention, the retaining member may be removably attached to the base. Thus, by removing the retaining member after forming the cultured tissue and the like, sufficient working space is secured on the culture surface, whereby the cultured tissue can be handled easily.

The tissue piece pinching device according to the present invention may also include a restriction mechanism that restricts movement of the retaining member in a direction apart from the culture surface in a state where the retaining member is depressing the tissue piece mounted on the culture surface. Thus, during culturing, even when a force is unexpectedly applied to the retaining member in a direction apart from the culture surface, the restriction mechanism restricts the movement of the retaining member in the direction apart from the culture surface, and the state in which the retaining member depresses the tissue piece is maintained. Thus, the tissue piece adhered to the culture surface is not peeled from the culture surface, and the tissue piece can be cultured smoothly.

A culture kit according to the present invention includes: a culture chamber that has an opening capable of receiving a tissue piece inserted therein; and either one of the above-described tissue piece pinching devices arranged in the culture chamber so that an end of the arm that is on the opposite side to the oscillating shaft faces the opening. Particularly, in the above-described culture kit, the culture chamber is preferably a flexible culture bag capable of being closed liquid-tight.

In the culture kit, either one of the above-described tissue piece pinching devices is arranged in the culture bag, and the end of the arm that is on the opposite side to the oscillating shaft faces the opening. Thus, since the arm that oscillates to separate the retaining member from the culture surface widens the inside of the culture bag, the working space for mounting the tissue piece on the culture surface and taking out the cultured tissue, which is the cultured tissue piece, can be secured, and an opening area of the opening can be made large. Also, since the culture kit includes either one of the above-described tissue piece pinching devices, the same effect as that of the above-described tissue piece pinching device can be obtained.

In the culture kit according to the present invention, the culture bag may have gas permeability and liquid impermeability. In this way, without leaking the liquid culture medium in the culture bag, the peripheral gas component can be taken into the culture bag, whereby culturing can be performed preferably. Here, although the material having gas permeability and liquid impermeability is not limited, fluorine resin such as polytetrafluoroethylene or silicon resin may be cited. These may be a solid body, and may be formed with minute holes (for example, holes with a diameter of several to several tens of micrometers).

### BRIEF DESCRIPTION OF THE DRAWINGS

[FIG. 1] FIG. 1 is a schematic perspective view of a culture kit 10 seen from a predetermined direction.
[FIG. 2] FIG. 2 is a schematic perspective view of a tissue piece pinching device 30 seen from a predetermined direction.
[FIG. 3] FIG. 3 is a schematic perspective view of the tissue piece pinching device 30 seen from another direction.
[FIG. 4] FIG. 4 is an explanatory view of the tissue piece pinching device 30 seen from the front.
[FIG. 5] FIG. 5 is a cross-sectional view along A-A of FIG. 2.
[FIG. 6] FIG. 6 is a schematic perspective view of a back surface of a base 31.
[FIG. 7] FIG. 7 is a vertical cross-sectional view showing a usage state of the culture kit 10.
[FIG. 8] FIG. 8 is a lateral cross-sectional view showing a usage state of the culture kit 10.
[FIG. 9] FIG. 9 is a vertical cross-sectional view of another tissue piece pinching device.

### BEST MODES FOR CARRYING OUT THE INVENTION

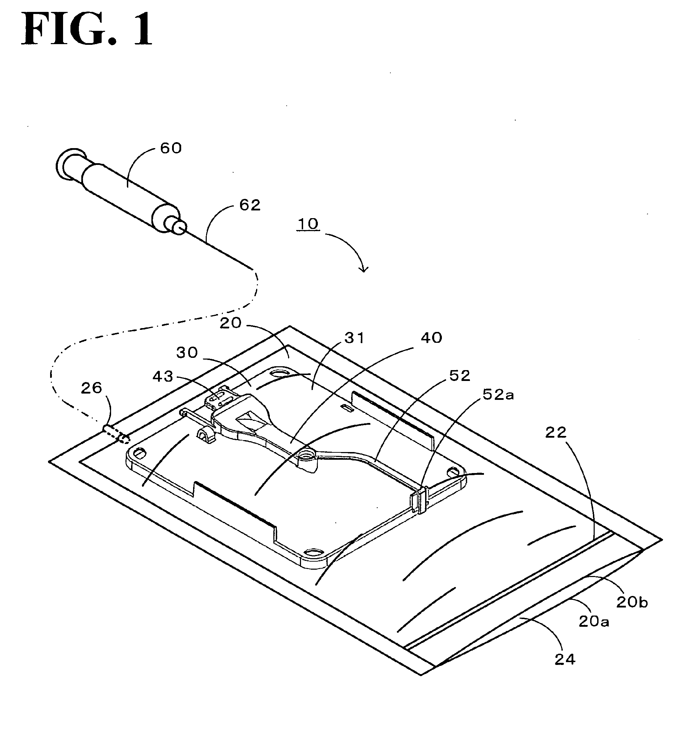

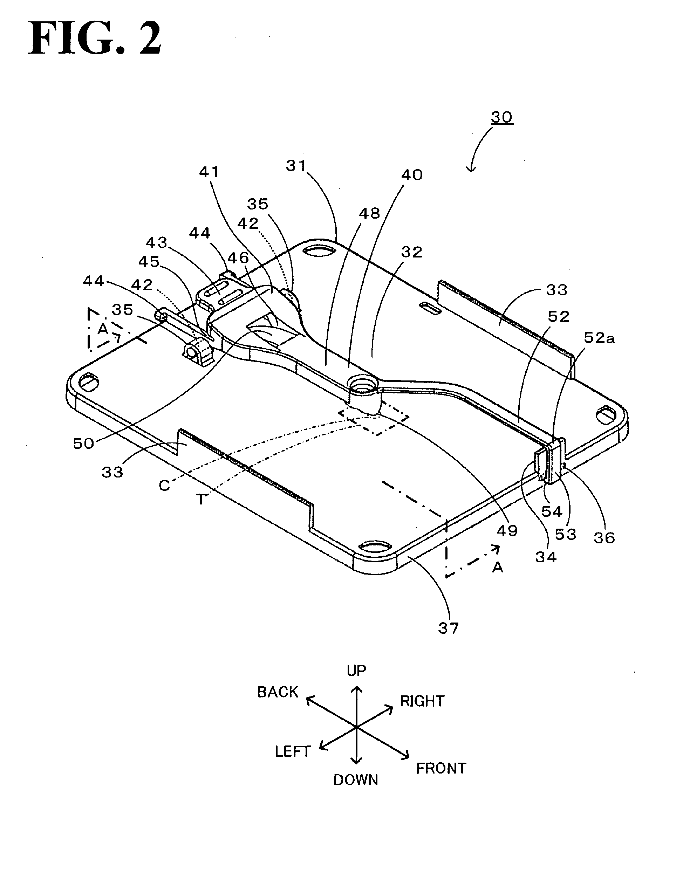

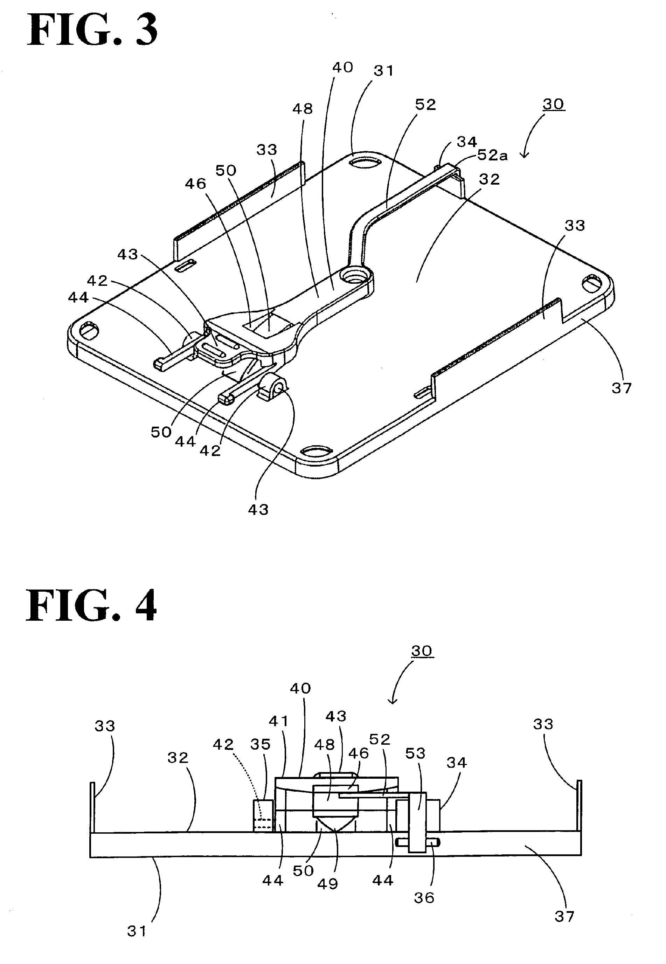

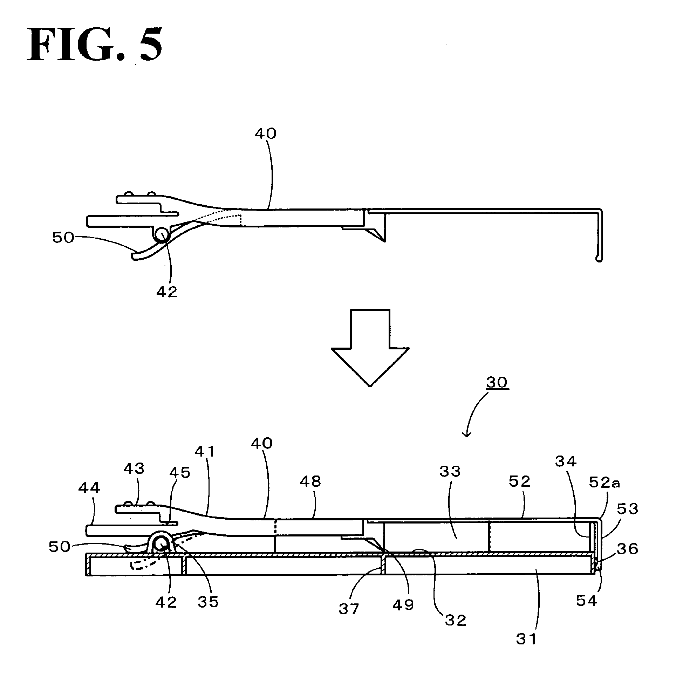

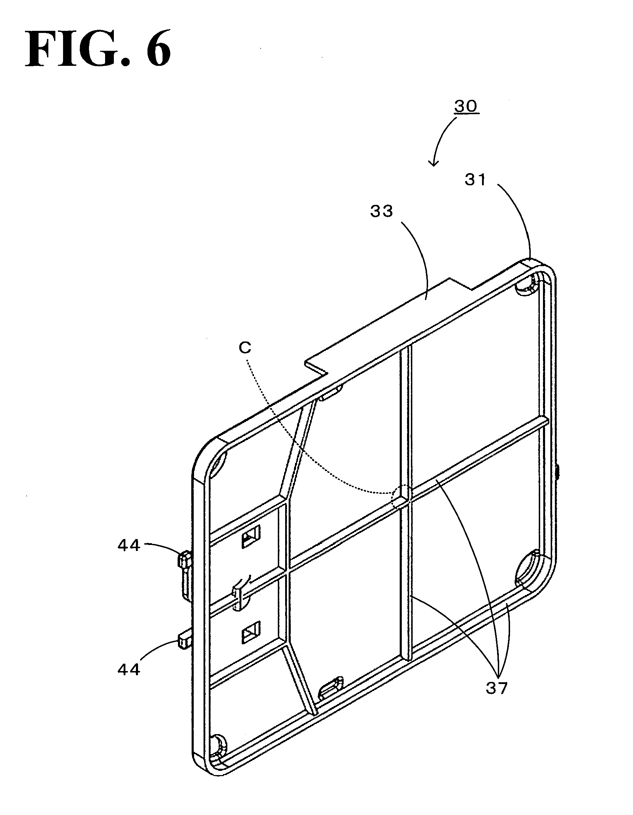

Hereinafter, the present invention will be explained with reference to the drawings. FIG. 1 is a schematic perspective view of a culture kit 10 of an embodiment of the present invention seen from a predetermined direction. FIG. 2 is a schematic perspective view of a tissue piece pinching device 30 seen from a predetermined direction. FIG. 3 is a schematic perspective view of the tissue piece pinching device 30 seen from another direction. FIG. 4 is an explanatory view of the tissue piece pinching device 30 seen from the front. FIG. 5 is a cross-sectional view along A-A of FIG. 2. FIG. 6 is a schematic perspective view of a back surface of a base 31.

The culture kit 10 according to the present embodiment includes a culture bag 20 capable of culturing a cell or a tissue piece, and the tissue piece pinching device 30 arranged in the culture bag 20, as shown in FIG. 1. In FIG. 1, the tissue piece pinching device 30 should normally be outlined by a hidden line (dotted line) since the tissue piece pinching device 30 is arranged inside the culture bag 20. However, the device 30 is outlined by a dotted line for the sake of convenience.

The culture bag 20 has its peripheral three sides welded by heating, with a lower surface 20a and an upper surface 20b made of white translucent fluorine resin having gas permeability and liquid impermeability being superimposed, and includes an opening 24 capable of being closed liquid-tight by a zipper 22. This culture bag 20 includes, on the opposite side to the opening 24, a syringe insertion 26. The syringe insertion 26 has a rubber plug fitted liquid-tightly into a tubular hole that makes the inside and the outside of the culture bag 20 communicate with each other. A needle 62 of a syringe 60 is inserted into the syringe insertion 26 when supplying or discharging a liquid culture medium to or from the culture bag 20. Also, since the hole after the needle 62 inserted in the syringe insertion 26 is taken out is closed up by the restoring force of the rubber, the syringe insertion 26 maintains a liquid-tight state again. In place of or in addition to liquid-tightly closing the opening 22 by the zipper 22, heat sealing or the like may be used for liquid-tight closing.

As shown in FIG. 2 to FIG 5, the tissue piece pinching device 30 includes a base 31 that has a culture surface 32 for mounting a tissue piece T (see FIG. 2) of a several millimeters square, and an arm 40 oscillatably attached to the base 31.

The base 31 is formed by molding translucent synthetic resin into a square shape, and includes the culture surface 32 added with a hydrophilic property by plasma treatment. Since the culture surface 32 has a hydrophilic property, adhesion-dependent cells and tissue pieces are easily adhered thereto. Also, the base 31 has, on its right and left sides, side walls 33 serving as projections to surround the culture surface 32. The side walls 33 support the upper surface 20b of the culture bag 20 from the inside, and prevent the culture bag 20 from contacting the cultured tissue on the culture surface 32. Further, the base 31 is provided with a front wall 34 (that corresponds to a supporting member of the present invention) at the front, and a stay 35 that supports pins 42 each serving as an oscillating shaft of the arm 40 at the back. Furthermore, below the front wall 34, a linear protrusion 36 engageable with a hook 54 of the arm 40 is provided. Meanwhile, on a back surface of the base 31, as shown in FIG. 6, reinforcing ribs 37 are provided. The reinforcing ribs 37 are formed at the whole circumference of the back surface, and are also formed so as to reinforce the periphery of the stay 35 and a mounting area of the culture surface 32 on which the tissue piece T is mounted. In the present embodiment, the reinforcing ribs 37 intersect at the back side of the center C (see FIG. 2 and FIG. 6) of the mounting area. Here, since the base 31 is made of white translucent synthetic resin, the intersection of the reinforcing ribs 37 can be seen through when the culture surface 32 is viewed from above. The intersection indicates the center C (a position of a retaining member on the culture surface) of the mounting area, and indicates where to mount the tissue piece T.

As shown in FIG. 2 to FIG. 5, the arm 40 includes a base end portion 41 having a large width positioned at a rear portion of the arm 40, an intermediate portion 48 having an intermediate width and extending toward the front from the base end portion 41, and an extended portion 52 having the smallest width and further extending towards the front from the intermediate portion 48. These portions are integrally formed by synthetic resin such as polyethylene, polypropylene, and polycarbonate. Every portion of the arm 40 has its width in the horizontal direction smaller than that of the base 31. The term "width" in this specification means the length in the horizontal direction of FIG. 2.

The base end portion 41 is provided with the pins 42 each of which projects on both sides in the horizontal direction, an operating button 43 (that corresponds to an arm operating member of the present invention) provided on an upper side behind the pins 42, and a pair of leg portions 44 formed so as to extend behind the pins 42. The pins 42 are supported by the stay 35 erected on the culture surface 35, whereby the arm 40 can oscillate by using the pins 42 as shafts. The operating button 43 is a button to be depressed by an operator when operating the arm 40, and is provided on the opposite side to a retaining portion 49 with the pins 42 interposed therebetween. Also, the pair of leg portions 44 leads to the operating button 43 via a notch 45. The pins 42 are disengaged from the stay 35 by moving (pinching) the leg portions 44 inward, whereby the arm 40 can be removed from the base 31. Further, at a substantial center of the base end portion 41, a square hole 46 that penetrates therethrough in the vertical direction is provided.

The intermediate portion 48 includes the retaining portion 49 provided at a position capable of retaining the tissue piece T mounted on the culture surface 32, and a leaf spring 50 (that corresponds to an urging member of the present invention) abutting the culture surface 32 through the square hole 46 of the base end portion 41. The retaining portion 49 is formed in a shape that is downwardly tapered so as to point-contact the tissue piece T. The leaf spring 50 is formed integrally with the arm 40. In a state where the pin 42 of the arm 40 is not supported by the stay 35 and is free, the leaf spring 50 is curved into a mound shape as shown in the upper section of FIG. 5. In a state where the pin 42 is supported by the stay 35, the mound shape is pressed from above as shown in the lower drawing of FIG. 5, whereby the leaf spring 50 urges the retaining portion 49 towards the culture surface 32.

As shown in FIG. 2, the extended portion 52 diagonally extends towards the front from the intermediate portion 48 near the retaining portion 49, and further extends straight towards the front, and has an inflected piece 53 at a tip 52a. The inflected piece 53 is formed by downwardly inflecting the extended portion 52 substantially at a right angle. Also, at the tip of the inflected piece 53, a hook 54 that can be engaged with the linear protrusion 36 provided on the base 31 is provided. In a state where the hook 54 is engaged with the linear protrusion 36, a top end of the front wall 34 provided on the base 31 substantially contacts a lower surface of the extended portion 52. Also, the tissue piece pinching device 30 is, as shown in FIG. 1, arranged so that the tip 52a of the extended portion 52 of the arm 40 is directed to the opening 24 of the culture bag 20.

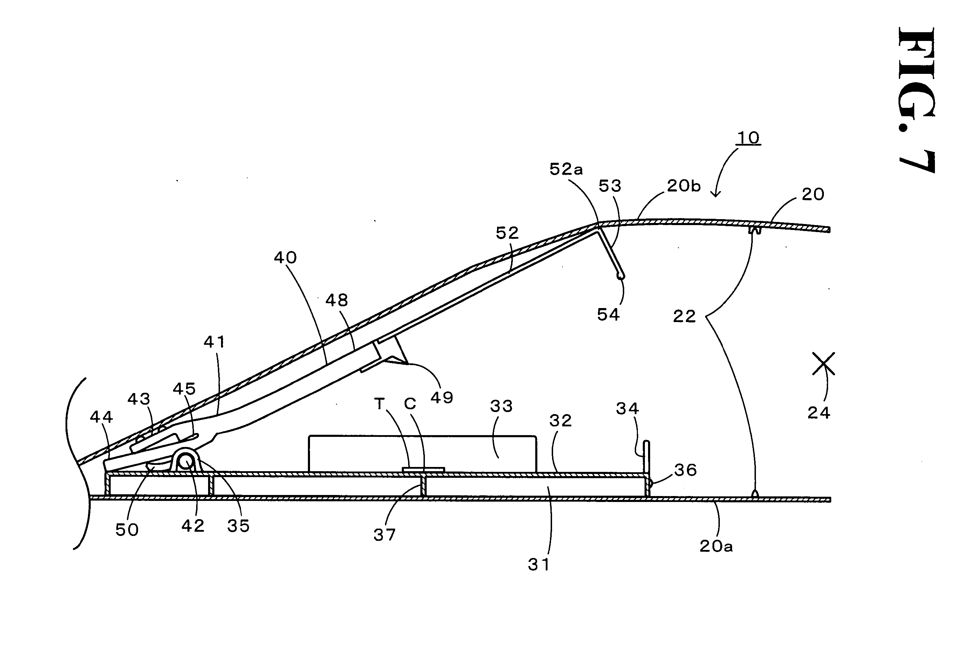

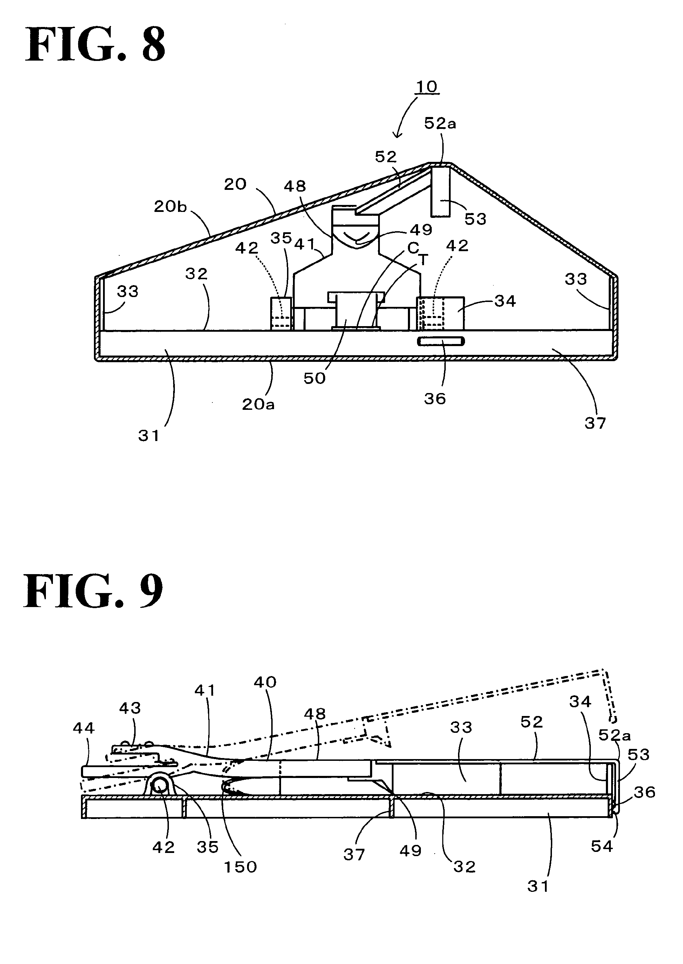

Next, a usage example of the culture kit 10 according to the present embodiment will be explained with reference to FIG. 7 and FIG. 8. FIG. 7 and FIG. 8 are a vertical cross-sectional view and a lateral cross-sectional view, respectively, showing a state where the operating button 43 is depressed.

First, the operator places the culture kit 10 shown in FIG. 1 on a platform so that the base 31 of the tissue piece pinching device 30 is substantially horizontal, and then depresses the operating button 43 of the arm 40 with one finger with the zipper 22 of the culture bag 20 open. Then, as shown in FIG. 7 and FIG. 8, a force for oscillating the arm 40 to separate the retaining portion 49 upward from the culture surface 32 around the pin 42 acts against the urging force of the leaf spring 50. Consequently, the tip 52a of the extended portion 52 ascends to a predetermined position while resisting the urging force of the leaf spring 50. Such predetermined position is determined by the downward traveling distance of the operating button 43 or the length from the pin 42 to the tip 52a of the extended portion 52, for example. Since the lower surface 20a of the culture bag 20 is retained by the base 31 and the upper surface 20b is raised upward by the tip 52a of the extended portion 52, the opening 24 of the culture bag 20 is widely opened. Also, since the width of the arm 40 is smaller than that of the base 31, the upper space of the culture surface 32 is not occupied by the arm 40, and a sufficiently wide work space is provided in the culture bag 20.

Next, while depressing the operating button 43, the operator mounts the tissue piece T (a periosteum tissue piece of a several millimeters square in this example) at the center C of the mounting area. At this time, the intersection of the reinforcing ribs 37 of the base 31 indicates the center C of the mounting area. Since this intersection can be seen through from above the base 31, the operator can securely mount the tissue piece T on the center C of the mounting area. Also, since the front wall 34 is provided at a position deviated from the front surface of the center C of the tissue piece mounting area, mounting of the tissue piece T is not disturbed by the front wall 34.

Next, the operator releases the depression of the operating button 43. Then, by the urging force of the leaf spring 50, the retaining portion 49 of the arm 40 depresses the tissue piece T mounted on the culture surface 32. At this time, since the retaining portion 49 is formed in a shape that is downwardly tapered, the retaining portion 49 point-contacts the tissue piece T. Consequently, the tissue piece T can be securely fixed to the culture surface 32 compared with the case of plane contact. Then, the operator gently depresses the tip 52a of the extended portion 52 so that the hook 54 of the arm 40 is engaged with the linear protrusion 36 (this operation is unnecessary when the hook 54 of the arm 40 is engaged with the linear protrusion 36 by the urging force of the leaf spring 50). Consequently, even when a slight force is unexpectedly applied in a direction (a direction in which the retaining portion moves upward) of depressing the operating button 43, since the hook 54 is engaged with the linear protrusion 36, the retaining portion 49 can be prevented from being separated upward from the culture surface 32, and the tissue piece can be prevented from being peeled. Also, even when a slight force is unexpectedly applied in a direction of depressing the extended portion 52, the front wall 34 blocks the downward movement of the extended portion 52, whereby the retaining portion 49 can be prevented from strongly depressing the culture surface 32. Thereafter, the operator closes the zipper 22 of the opening 24 of the culture bag 20. Then, the inside of the culture bag 20 becomes liquid-tight.

Next, the operator sticks the needle 62 of the syringe 60 shown in FIG. 1 into the syringe insertion 26, and injects an adequate amount of a liquid culture medium.

Next, the operator takes the needle 62 of the syringe 60 out from the syringe insertion 26. Then, the syringe insertion 26 will have its hole that was generated with the needle 62 stuck thereinto closed by the restoring force of the rubber. Thus, the inside of the culture bag 20 is kept liquid-tight. Thereafter, the culture bag 20 is placed into a not shown carbon dioxide gas incubator to start culturing the tissue piece. Since the culture bag 20 has gas permeability, the gas undergone a component adjustment in the carbon dioxide gas incubator penetrates to the inside of the culture bag 20, dissolves into the liquid culture medium, and is supplied to the tissue piece T. Also, since the tissue piece T is depressed in point contact by the retaining portion 49, the culture medium favorably penetrates. During culturing, replacement of the culture medium is performed as needed. In such a case, the syringe 60 is used to discharge the used liquid culture medium, and a new liquid culture medium is injected.

After culturing is completed, the used liquid culture medium is discharged by using the syringe 60. Then, the zipper 22 is opened, and the tissue piece pinching device is taken out from the culture bag 20. After cleaning the tissue piece pinching device with cleaning liquid, the arm 40 is removed from the base 31 by pinching the leg portions 44. Then, the cultured tissue is collected. By removing the arm 40 from the base 31 in such a way, the operator can easily access the cultured tissue formed on the culture surface. Thus, the operability becomes favorable.

Moreover, without taking out the tissue piece pinching device from the culture bag, the cultured tissue may be removed from the culture surface 32 and taken out to the outside of the culture bag 20 by opening the zipper 22 of the culture bag 20, depressing the operating button 43, and separating the retaining portion 49 from the culture surface 32. In this case, after the culture is completed, the used liquid culture medium is discharged by using the syringe 60. Thereafter, likewise, by using the syringe 60, cleaning liquid is injected. Then, after discharging and injecting of the cleaning liquid are repeated for several times, the zipper 22 of the culture bag 20 is opened in a state where the cleaning liquid is discharged, the operating button 43 is depressed, and the retaining portion 49 is separated from the culture surface 32. Consequently, the opening 24 of the culture bag 20 is widely opened, and the cultured tissue, which is the cultured tissue piece T, is removed from the culture surface 32 and taken out to the outside of the culture bag 20. In this case, since the width of the arm 40 is smaller than that of the base 31, the upper space of the culture surface 32 is not occupied by the arm 40, and a sufficiently wide work space is provided in the culture bag 20.

To collect the culture medium as a cultured cell suspension, following the next steps will make the collection easy. After the culturing is completed, the used liquid culture medium is discharged by using the syringe 60. Thereafter, likewise, by using the syringe 60, the cleaning liquid is injected. Then, after discharging and injecting of the cleaning liquid are repeated for several times, cell dispersion liquid is injected into the culture bag 20 by using the syringe 60. After a predetermined time has passed, a terminator for cell dispersion is injected by using the syringe 60, and the cell dispersed with such liquid solution is sucked by using the syringe 60, whereby the cultured cell suspension is collected.

According to the above-described embodiment, when opening a space between the culture surface 32 and the retaining portion 49, the arm 40 is oscillated. Therefore, the operability becomes favorable. Also, since the width of the arm 40 is smaller than that of the base 31, a sufficiently wide working space is provided when holding the tissue piece T in the tissue piece pinching device 30, and when taking out the cultured tissue, which is the cultured tissue piece T, from the tissue piece pinching device 30, and the operability becomes favorable. Also, during culturing the tissue piece T, the tissue piece T is depressed against the culture surface 32 by the retaining portion 49 urged by the leaf spring 50, whereby the tissue piece T adheres to the culture surface 32, and becomes easy to grow. Also, movement of the arm 40 can be easily operated from the outside of the culture bag 20. Further, since the leaf spring 50 is formed integrally with the arm 40, the number of components is smaller compared with a case where these components are formed separately, whereby the assembling process is simplified. Still further, although the tissue piece T is depressed against the culture surface 32 by the retaining portion 49 during culturing, the front wall 34 prevents the extended portion 52 from moving downward even when a force is unexpectedly applied to the extended portion 52 from above, whereby the retaining portion 49 never depresses the tissue piece T too hard against the culture surface 32. Also, since the front surface 34 is provided in a position deviated from the direct-front surface of the retaining portion 49, the front wall 34 will never be disturbing, for example, when mounting the tissue piece T directly below the retaining portion 49. Further, since the retaining portion 49 point-contacts the tissue piece T, compared with the retaining portion 49 formed in such a shape that plane-contacts the tissue piece T, the tissue piece T can be securely fixed to the culture surface 32, and penetration (penetrativity) of the culture medium to the tissue piece T becomes favorable. Still further, since the side walls 33 surround the periphery of the culture surface 32, even in a case where the culture bag 20 deflates when the culture medium is taken out from the culture bag 20 for replacing the culture medium, the side walls 33 prevent the tissue piece T from contacting the upper surface 20b of the culture bag 20.

The present invention is not limited to the embodiment described above, and may be implemented in other embodiments within the technical scope of the present invention.

For example, in the above-described embodiment, the arm 40 is provided with the extended portion 52, however, the extended portion 52 may be omitted. In this case, since the culture bag 20 is lifted over the retaining portion 49 of the arm 40, the lifting amount of the culture bag 20 becomes smaller compared with the above-described embodiment. Still, almost the same effect as in the above-described embodiment can be obtained.

In the above-described embodiment, the leaf spring 50 urges the retaining portion 49 of the arm 40 towards the culture surface 32. However, as shown in FIG. 9, a leaf spring 150 formed integrally with the arm 40 may urge the retaining portion 49 so as to separate the retaining portion 49 from the culture surface 32. In FIG. 9, the same reference numerals are denoted for the same components as in the above-described embodiment. In this case, in a state where the operator does not operate the operating button 43, the retaining portion 49 is separated upward from the culture surface 32 as shown by the dashed lines in FIG. 9. Therefore, similarly to the state (see FIG. 7) where the operating button 43 is depressed in the above-described embodiment, the opening 24 of the culture bag 20 can be widely opened, and a sufficiently wide work space can be obtained when mounting the tissue piece T directly below the retaining portion 49 and when taking out the cultured tissue, which is the cultured tissue piece T, from directly below the retaining portion 49. On the other hand, in order to fix the tissue piece T on the culture surface 32 by the retaining portion 49, the operator pushes up the operating button 43 or pushes down a center portion 48 and the extended portion 52 to make the hook 54 of the arm 40 be engaged with the linear protrusion 36 of the base 31, whereby the tissue piece T is pressed against the culture surface 32 by the retaining portion 49 as shown by the solid lines in FIG. 9. In this case, the engagement of the linear protrusion 36 and the hook 54 is designed so as not to be disengaged by the urging force of the leaf spring 150 alone. In order to separate the retaining portion 49 upward from the culture surface 32 again, the operator depresses the operating button 43 or disengages the hook 54 from the linear protrusion 36. Adopting the structure of FIG. 9 can provide almost the same effect as in the above-described embodiment.

In the above-described embodiment, the hook 54 is provided on the inflected piece 53 as an engaging material of the present invention, and the linear protrusion 36 is provided on the base 31 as a to-be-engaged material. However, the hook 54 may be made to be stuck at the bottom surface of the base 31, and the linear protrusion 36 may be omitted. Alternatively, a substantially semispherical protrusion may be provided instead of the hook 54 on the inflected piece 53, and a hole in which the protrusion is fit may be provided on the base 31 instead of the linear protrusion 36. On the contrary, a substantially semispherical protrusion may be provided on the base 31, and a hole in which the protrusion is fit may be provided on the inflected piece 53. Any other structure may be applied as long as the structure is detachably engaged as described above.

In the above-described embodiment, the retaining portion 49 is formed in a shape that is downwardly tapered so that the retaining portion 49 point-contacts the tissue piece T. However, the retaining portion 49 may be formed into such a shape that the retaining portion 49 line-contacts the tissue piece T. In this case, compared with the retaining portion 49 formed so as to plane-contact the tissue piece T, the tissue piece T can be securely fixed to the culture surface 32, and the penetration of the culture medium to the tissue piece T becomes preferable. However, the present invention is not intended to exclude such shapes that the retaining portion 49 plane-contacts the tissue piece T.

In the above-described embodiment, the culture kit 10 is formed of the culture bag 20 and the tissue piece pinching device 30. However, the syringe 60 and a liquid culture medium necessary for culturing may be included in the culture kit 10.

In the above-described embodiment, an explanation is given that a hydrophilic property is added to the culture surface 32. However, the present invention is not limited thereto. For example, by making cells intake magnetic particulates, adhesion-dependent cells can be cultured on a non-adherent culture surface (see Japanese Patent Application Publication No. JP-A-2004-254519). Therefore, the culture surface does not necessarily have to have a hydrophilic property. Also, by composing the culture surface 32 with a temperature-responsive polymer, the culture surface 32 may be switched between a hydrophilic property and a hydrophobic property through temperature control, whereby the adhesiveness of cells can be controlled.

This application is based upon and claims the priority of Japanese Patent Application No. 2006-312136 filed on November 17th, 2006, and Japanese Patent Application No. 2006-312137 filed on November 17th, 2006, and the entire content thereof is included in this specification by reference.

### INDUSTRIAL APPLICABILITY

The present invention is applicable to a field of, for example, regeneration medicine.

## Claims

1. A tissue piece pinching device capable of being arranged in a culture chamber, the device comprising:
a base having a culture surface for mounting a tissue piece;
an arm oscillatably attached to the base; and
a retaining member, as a part of the arm, provided in a position capable of retaining the tissue piece mounted on the culture surface.

2. The tissue piece pinching device according to claim 1, wherein the width of the arm is smaller than the width of the base.

3. The tissue piece pinching device according to claim 1 or 2, further comprising:
an urging member that urges the retaining member towards the culture surface; and
an arm operating member that is provided on the opposite side to the retaining member, with an oscillating shaft that is used when the arm oscillates interposed therebetween, and that separates, when depressed by an external force, the retaining member from the culture surface while resisting the urging member.

4. The tissue piece pinching device according to claim 3, wherein the urging member is integrally formed with the arm.

5. The tissue piece pinching device according to any one of claims 1 to 4, further comprising:
an extended member that extends from a position of the retaining member, as a part of the arm, in a direction opposite to the oscillating shaft used when the arm oscillates.

6. The tissue piece pinching device according to claim 5, wherein the base comprises a supporting member at a position capable of contacting a lower surface of a tip end side of the extended member, the supporting member preventing the extended member from rotating downward to exceed a predetermine range around the oscillating shaft of the arm.

7. The tissue piece pinching device according to claim 6, wherein the supporting member is provided in a position deviated from the direct-front surface of the retaining member.

8. The tissue piece pinching device according to any one of claims 1 to 7, wherein the retaining member is formed in such a shape that point-contacts or line-contacts the tissue piece mounted on the culture surface.

9. The tissue piece pinching device according to any one of claims 1 to 8, wherein the base includes a projection at least at a part of the peripheral of the culture surface.

10. The tissue piece pinching device according to any one of claims 1 to 9, wherein the retaining member is detachably attached to the base.

11. The tissue piece pinching device according to any one of claims 1 to 10, further comprising:
a restriction mechanism that restricts movement of the retaining member in a direction apart from the culture surface in a state where the retaining member is depressing the tissue piece mounted on the culture surface.

12. A culture kit comprising:
a culture chamber that has an opening capable of receiving a tissue piece inserted therein; and
a tissue piece pinching device according to any one of claims 1 to 11, the device being arranged in the culture chamber so that an end of the arm that is on the opposite side to the oscillating shaft faces the opening.

13. The culture kit according to claim 12, wherein the culture chamber is a flexible culture bag capable of being closed liquid-tight.
